# EUROPÄISCHE PATENTSCHRIFT

(11) **EP 1 475 077 B1**
(45) Veröffentlichungstag und Bekanntmachung des Hinweises auf die Patenterteilung: **02.08.2006**
(21) Anmeldenummer: 04010428.3
(22) Anmeldetag: 03.05.2004
(51) Int. Cl.: A61Q 15/00, A61K 8/03, A61K 8/891, A61K 8/67, A61K 8/34, A61K 8/26, A61K 8/27, A61K 8/28, A61K 8/29, A61K 8/20, A61K 8/39, A61K 8/73

(54) **Zweiphasige flüssige Antitranspirant-Zusammensetzungen**
Double phase liquid antitranspirant compositions
Compositions anti-transpirantes liquides à deux phases

(30) Priorität: 09.05.2003 DE 10321138
(43) Veröffentlichungstag der Anmeldung: 10.11.2004
(73) Patentinhaber: Henkel Kommanditgesellschaft auf Aktien, 40589 Düsseldorf (DE)
(72) Erfinder: Banowski, Bernhard, 40597 Düsseldorf (DE); Wadle, Armin, 40699 Erkrath (DE)

(56) Entgegenhaltungen:
- EP-A- 0 370 856
- WO-A-20/04055151
- US-A- 3 533 955
- US-A- 5 468 496
- US-A1- 2002 098 209
- PATENT ABSTRACTS OF JAPAN Bd. 0092, Nr. 18 (C-301), 5. September 1985 (1985-09-05) & JP 60 078907 A (KOBAYASHI KOOSEE:KK), 4. Mai 1985 (1985-05-04)
- PATENT ABSTRACTS OF JAPAN Bd. 0122, Nr. 37 (C-509), 6. Juli 1988 (1988-07-06) & JP 63 027409 A (SHISEIDO CO LTD), 5. Februar 1988 (1988-02-05)

## Beschreibung

Gegenstand der vorliegenden Erfindung sind kosmetische Rezepturen für flüssige, zweiphasige Antitranspirant-Zusammensetzungen.

Erfindungsgemäß bedeutet das Merkmal "zweiphasig", dass die Formulierung eine wässrige und eine Ölphase aufweist, die im Ruhestand als zwei durch eine Phasengrenze getrennte Flüssigkeitsschichten vorliegen, aber vor dem Gebrauch durch kurzes Schütteln des Behälters zu einer ÖI-in-Wasser-Emulsion oder einer Wasser-in-ÖI-Emulsion vermischt werden.

Um ein ansprechendes optisches Erscheinungsbild der Formulierung zu erzielen, ist es wünschenswert, dass sich beide Phasen schnell und möglichst vollständig wieder entmischen, sobald sie im Ruhezustand sind. Die Zeit bis zur vollständigen Entmischung reicht dabei von wenigen Sekunden bis zu 24 Stunden.

Zweiphasige kosmetische Zusammensetzungen mit nicht dauerhaft emulgierter Öl- und Wasserphase sind im Stand der Technik bekannt.US 2002/098209 offenbart flüssige, zweiphasige Zusammensetzungen, die geeignet sind für Kosmetik und Pharmazie. Es zeigt sich, dass insbesondere die Faktoren Emulgatormenge und -typ, Verhältnis zwischen ÖI- und Wasserphase, Elektrolytgehalt, Verdickergehalt und ggf. die Anwesenheit von Alkoholen die Eigenschaften derartiger Zusammensetzungen, vor allem die Geschwindigkeit der Phasentrennung, beeinflussen.
Über die Zweiphasigkeit lassen sich verschiedene optische Effekte einstellen. Das Sichtbarmachen der Ölphase unterstreicht optisch für den Verbraucher den pflegenden Effekt derartiger Zusammensetzungen. Durch die Wahl öllöslicher bzw. wasserlöslicher Farbstoffe kann die optische Unterscheidung der beiden Phasen betont werden. Bei der kurzzeitigen Emulgierung bilden sich Mischfarben, die sich bei der anschließenden Phasentrennung durch das Entmischen effektvoll verändern. Die Auswahl der einzelnen Bestandteile der Zusammensetzung kann auch so erfolgen, dass während des Entmischungsvorgangs für eine gewisse Zeit eine intermediäre dritte Emulsionsschicht als Phasengrenze zwischen ÖI- und Wasserphase bestehen bleibt. Ästhetisch weniger ansprechende Phänomene, z.B. Tropfen- oder Schlierenbildung an der Innenwand des Aufbewahrungsbehälters, sollten durch geeignete Abstimmung der einzelnen Bestandteile aufeinander vermieden werden.

Antitranspirant-Zusammensetzungen enthalten adstringierende Aluminium-, Zink-, Zirkon- oder Titanverbindungen in höheren Mengen. Auf diesen vorgegebenen Elektrolytgehalt muss der Fachmann die Wahl der übrigen Komponenten abstimmen, um ein den Konsumenten zufriedenstellendes Produkt herzustellen.

Die im Stand der Technik bekannten Zusammensetzungen weisen in vielerlei Hinsicht noch Nachteile auf, die durch die vorliegende Erfindung überwunden werden.

Aufgabe der vorliegenden Erfindung war es, eine Applikationsform für Deodorant- oder Antitranspirant-Zusammensetzungen, die vorzugsweise für die Anwendung in der Achselhöhle vorgesehen sind, zu entwickeln, bei der die Zusammensetzung am ursprünglichen Applikationsort verbleibt, ohne sichtbare Rückstände zu hinterlassen. Eine weitere Aufgabe war es, eine Zusammensetzung bereitzustellen, die ein frisches, angenehmes Hautgefühl hinterlässt. Eine weitere Aufgabe war es, eine Zusammensetzung bereitzustellen, die durch ihr Aussehen und ihre Konsistenz beim Auftragen einen pflegenden Eindruck erweckt.
Eine weitere Aufgabe war die Bereitstellung einer Zusammensetzung, mit der sich ansprechende optische Effekte erzielen lassen.

Die Probleme des Standes der Technik wurden überraschenderweise gelöst durch die nachfolgend dargelegten kosmetischen Zusammensetzungen.

Ein erster Gegenstand der vorliegenden Erfindung ist eine kosmetische Antitranspirant-Zusammensetzung, enthaltend eine Ölphase und eine Wasserphase, die im Ruhezustand nicht-emulgiert und durch eine Phasengrenze voneinander getrennt vorliegen, durch kurzzeitiges Schütteln emulgierbar sind, wobei die vollständige Entmischung im Ruhezustand innerhalb von 5 Sekunden bis 24 Stunden erfolgt, gekennzeichnet durch einen Gehalt an
a) 15 - 50 Gew.-% Ölphase, deren einzelne Komponenten miteinander eine klare Mischung bilden, enthaltend
   - mindestens ein kosmetisches, bei 25° C flüssiges Öl,
   - mindestens ein Parfümöl,
   - 0,01 - 2 Gew.-% eines nichtionischen, kationischen, zwitterionischen oder amphoteren Emulgators,
b) 50 - 85 Gew.-% wässrige Phase, enthaltend
   - 2 - 30 Gew.-%, mindestens einer Aluminium-, Zirkon-, Zink- oder TitanVerbindung mit transpirationshemmender Wirkung,
   - mindestens ein mit den transpirationshemmenden Verbindungen kompatibles organisches Polymer mit verdickender Wirkung,
   - mindestens ein wasserlösliches Polyol,
   - 0,5 - 10 Gew.-% Natrium- oder Kaliumchlorid,
wobei sich alle Mengenangaben in Gew.-% auf das Gewicht der Gesamtzusammensetzung beziehen.

Bei den erfindungsgemäßen Zusammensetzungen reicht die Zeit bis zur vollständigen Entmischung dabei von ca. 5 Sekunden bis zu 24 Stunden, bevorzugt 10 Sekunden bis 5 Stunden, besonders bevorzugt 30 Sekunden bis 30 Minuten.

Erfindungsgemäß geeignete nichtionische Emulgatoren sind beispielsweise
- alkoxylierte Fettsäurealkylester der Formel R¹CO-(OCH₂CHR²)ₓOR³, in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und x für Zahlen von 1 bis 20 steht,
- Anlagerungsprodukte von Ethylenoxid an Fettsäurealkanolamide und Fettamine,
- Fettsäure-N-alkylglucamide,
- C₈ - C₂₂-Alkylamin-N-oxide,
- Alkylpolygykoside entsprechend der allgemeinen Formel RO-(Z)ₓ wobei R für eine C₈- C₁₆-Alkylgruppe, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht.
Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5, bevorzugt 1,1 bis 2,0 besonders bevorzugt 1,1 bis 1,8 Zuckereinheiten. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weitere erfindungsgemäß geeignete nichtionische Emulgatoren sind ausgewählt aus den Anlagerungsprodukten von 4 bis 100 Ethylenoxid-Einheiten an gegebenenfalls gehärtete Mono-, Di- und Triglyceride von C₈₋₂₂-Fettsäuren, den Anlagerungsprodukten von 5 bis 40 Ethylenoxid-Einheiten an C₈₋₂₂-Fettalkohole sowie den Anlagerungsprodukten von 2 bis 50 Ethylenoxid-Einheiten und 2 bis 35 Propylenoxid-Einheiten an C₃-C₅₋Alkanole. Beispiele für ethoxylierte Mono-, Di- und Triglyceride von C₈₋₂₂-Fettsäuren mit 4 bis 60 Ethylenoxid-Einheiten sind hydriertes ethoxyliertes Castoröl (INCI-Bezeichnung z.B. PEG-40 Hydrogenated Castor Oil), Olivenölethoxylat (INCI-Bezeichnung: PEG-10 Olive Glycerides), Mandelölethoxylat, Nerzölethoxylat, Polyoxyethylenglycolcapryl-/caprinsäureglyceride, Polyoxyethylenglycerinmonolaurat und Polyoxyethylenglycolkokosfettsäureglyceride. Beispiele für geeignete ethoxylierte C₈₋₂₂-Fettalkohole sind Laureth-12, Laureth-23, Trideceth-8, Ceteareth-12, Ceteareth-15, Ceteareth-20, Ceteareth-30, Steareth-10, Steareth-15, Steareth-20, Steareth-30, Steareth-40, Oleth-10 oder Oleth-20. Beispiele für geeignete Polyethylenglycol-Polypropylenglycol-Mischether von C₃-C₅-Alkanolen sind die PEG-PPG-Addukte von 1-Propanol, 2-Propanol und isoPropanol, 1-Butanol, 2-Butanol, iso-Butanol und 1-Pentanol, 2-Pentanol und Amylalkohol mit 2 - 50, bevorzugt 4 - 40 Ethylenoxid-Einheiten und 2 - 35, bevorzugt 4 - 30 Propylenoxid-Einheiten, insbesondere PPG-28-Buteth-35, PPG-26-Buteth-26, PPG-5-Buteth-5, PPG-25-Buteth-25, PPG-5-Buteth-20, PPG-33-Buteth-45, PPG-20-Buteth-30 oder PPG-12-Buteth-16.
Geeignete nichtionische Emulgatoren sind weiterhin die Ethylenoxid-Addukte von linearen C₃-C₂₂-Alkoholen mit einer mittleren Anzahl der Ethylenoxid-Einheiten von 1-30. Bevorzugt geeignete nichtionische Emulgatoren sind Ethylenoxid-Addukte von verzweigten C₃₋C₂₈-Alkoholen, insbesondere von sogenannten Guerbet-Alkoholen, mit einer mittleren Anzahl von Ethylenoxid-Einheiten von 1-30.

Weitere bevorzugt geeignete nichtionische Emulgatoren sind Ethylenoxd-Propylenoxid-Mischaddukte von linearen C₃₋C₂₂-Alkoholen mit einer mittleren Anzahl an Ethylenoxid-Einheiten von 2-50, bevorzugt 4-40 und einer mittleren Anzahl an Propylenoxid-Einheiten von 2-35, bevorzugt 4-30.

Besonders bevorzugte nichtionische Emulgatoren sind Propylenoxid-Addukte von linearen C₃-C₂₂-Alkoholen. Die mittlere Anzahl der Propylenoxid-Einheiten beträgt 1-30, bevorzugt 5-25 und besonders bevorzugt 8-15. Geeignete propoxylierte Emulgatoren sind z. B. PPG-3-Myristylether (Witconol® APM), PPG-14-Butylether (Ucon Fluid® AP), PPG-15-Stearylether (Arlamol® E), PPG-9-Butylether (Breox® B25) und PPG-10-Butandiol (Macol® 57), wobei PPG-14-Butylether und PPG-15-Stearylether besonders bevorzugt sind.

Die erfindungsgemäßen Zusammensetzungen enthalten den nichtionischen Emulgatoren in Mengen von 0,01 - 2 Gew.-%, bevorzugt 0,05 - 1,5 Gew.-% und besonders bevorzugt 0,1 - 1,0 Gew.-%, jeweils bezogen auf die gesamte flüssige Zusammensetzung.

Als zwitterionische Emulgatoren werden solche oberflächenaktiven Verbindungen bezeichnet, die mindestens eine quartäre Ammoniumgruppe und mindestens eine -COO⁻ - oder -SO₃⁽⁻⁾ -Gruppe im Molekül tragen. Besonders geeignete zwitterionische Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugter zwitterionischer Emulgator ist das unter der INCI-Bezeichnung Cocamidopropyl Betaine bekannte Fettsäureamid-Derivat.

Erfindungsgemäß geeignet sind ebenfalls kationische Emulgatoren vom Typ der quartären Ammoniumverbindungen, der Esterquats und der Amidoamine. Bevorzugte quaternäre Ammoniumverbindungen sind Ammoniumhalögenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Emulgatoren weisen bevorzugt 10 bis 18 Kohlenstoffatome auf.
Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.
Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.
Esterquats sind erfindungsgemäß besonders bevorzugte kationische Emulgatoren.
In einer besonders bevorzugten Ausführungsform sind nur nichtionische Emulgatoren enthalten. Nichtschäumende Emulgatoren sind außerordentlich bevorzugt.
Die erfindungsgemäßen Zusammensetzungen enthalten die Emulgatoren in Mengen von 0,01 - 2 Gew.-%, bevorzugt 0,05 - 1,5 Gew.-% und besonders bevorzugt 0,1 - 1,0 Gew.-%, jeweils bezogen auf die gesamte flüssige Zusammensetzung.

Erfindungsgemäß geeignete bei 25° C flüssige kosmetische Ölkomponenten sind beispielsweise:
- Siliconöle, wobei insbesondere die linearen Siliconöle mit der INCI-Bezeichnung Dimethicone, Trisiloxane, Hexamethyldisiloxan bevorzugt sind, die cyclischen Siliconöle Octamethylcyclotetrasiloxan, Decamethylcyclopentasiloxan und Dodecamethylcyclohexasiloxan aber technisch auch geeignet sind,
- aliphatische Kohlenwasserstoffe, z. B. Paraffinöle und Isoparaffinöle, insbesondere Polyalphaolefine wie die Öle der Produktserie Nexbase, z.B. Hydrogenated Polydecene, weiterhin Polyisobuten, Polyethylen, z.B. der Rohstoff Performalene 400,
- alicyclische Kohlenwasserstoffe, die keine von Kohlenstoff und Wasserstoff verschiedenen Atome enthalten, z. B. substituierte Cyclohexane wie 1,3-Bis(2-ethylhexyl)cyclohexan (Cetiol® S),
- verzweigte primäre Alkohole, insbesondere Guerbetalkohole auf Basis von Fettalkoholen mit 6 bis 22, vorzugsweise 8 bis 10 Kohlenstoffatomen,
- die Ester von gesättigten oder ungesättigten, linearen oder verzweigten C₃-C₂₂-Fettsäuren oder den Dimeren ungesättigter C₁₂-C₂₂-Fettsäuren (Dimerfettsäuren) mit a) einwertigen linearen, verzweigten oder cyclischen C₂-C₂₂-Alkanolen, b) mehrwertigen linearen oder verzweigten C₂-C₆-Alkanolen, c) Polyglycerinen der Formel CH₂OH-CHOH-CH₂[-O-CH₂-CHOH-CH₂]ₙ-O-CH₂CHOH-CH₂OH mit n = 0 - 8, oder d) den Dicarbonsäure- und Tricarbonsäureestern von linearen und verzweigten C₂-C₁₀-Alkanolen, wobei als bevorzugte Komponenten a) die Ester von linearen C₆₋C₂₂-Fettsäuren mit linearen C₆-C₂₂-Fettalkoholen, sogenannte Wachsester, sofern diese bei 25° C flüssig sind, wie beispielsweise Jojobaöl, weiterhin die flüssigen Mono-/Di-/Triglyceridmischungen auf Basis von C₆-C₁₈-Fettsäuren sowie pflanzliche Öle und als bevorzugte Komponente d) Citronensäuretriethylester zu nennen sind,
- Ester von linearen und verzweigten C₁₂-C₂₂-Alkanolen ein- und mehrwertiger C₂-C₇₋Hydroxycarbonsäuren, die aromatisch sein können, insbesondere Benzoesäure (z.B. Finsolv® TN),
- Dialkylether, z. B. Dioctylether (Cetiol® OE),
- Ringöffnungsprodukte von epoxidierten Fettsäureestern mit Polyolen,
- sowie symmetrische, unsymmetrische oder cyclische Ester der Kohlensäure mit linearen und verzweigten C₈-C₁₈-Alkanolen, z. B. Cetiol® CC.

Die genannten Öle können sowohl einzeln als auch in Mischungen eingesetzt werden. Beim Einsatz von Mischungen ist darauf zu achten, dass die einzelnen Öle miteinander eine klare Mischung bilden. Schnelltrocknende Öle sind bevorzugt.
Die erfindungsgemäßen Zusammensetzungen enthalten die bei 25° C flüssige Ölkomponente in Mengen von 10 - 49 Gew.-%, bevorzugt 12 - 30 Gew.-%, besonders bevorzugt 15 - 25 Gew.-% und außerordentlich bevorzugt 18 - 22 Gew.-%, jeweils bezogen auf die gesamte flüssige Zusammensetzung.

Erfindungsgemäß geeignete Parfümöle sind zum Beispiel Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 - 18 C-Atomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.
Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl.

Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.
Die erfindungsgemäßen Zusammensetzungen enthalten das Parfümöl in Mengen von 0,01 - 2 Gew.-%, bevorzugt 0,1 - 1,5 Gew.-%, jeweils bezogen auf die gesamte flüssige Zusammensetzung.

Als Antitranspirant-Wirkstoffe eignen sich erfindungsgemäß wasserlösliche adstringierende oder einweißkoagulierende metallische Salze, insbesondere anorganische und organische Salze des Aluminiums, Zirkoniums, Zinks und Titans sowie beliebige Mischungen dieser Salze. Erfindungsgemäß wird unter Wasserlöslichkeit eine Löslichkeit von wenigstens 4 g Aktivsubstanz pro 100 g Lösung bei 20 °C verstanden. Erfindungsgemäß verwendbar sind beispielsweise Alaun (KAl(SO₄)₂ ·12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-ZirkoniumChlorohydrat, Zinkchlorid, Zinksulfocarbolat, Zinksulfat, Zirkoniumchlorohydrat, Aluminium-Zirkonium-Chlorohydrat-Glycin-Komplexe und Komplexe von basischen Aluminiumchloriden mit Propylenglycol oder Polyethylenglycol. Bevorzugt enthalten die flüssigen Wirkstoffzubereitungen ein adstringierendes Aluminiumsalz, insbesondere Aluminiumchlorohydrat, und/ oder eine Aluminium-Zirkonium-Verbindung. Aluminiumchlorohydrate werden beispielsweise pulverförmig als Micro Dry® Ultrafine oder in aktivierter Form als Reach® 501 oder Reach® 103 von Reheis sowie in Form wäßriger Lösungen als Locron® L von Clariant oder als Chlorhydrol® von Reheis vertrieben. Unter der Bezeichnung Reach® 301 wird ein Aluminiumsesquichlorohydrat von Reheis angeboten. Auch die Verwendung von Aluminium-Zirkonium-Tri- oder Tetrachlorohydrex-Glycin-Komplexen, die beispielsweise von Reheis unter der Bezeichnung Rezal® 36G im Handel sind, ist erfindungsgemäß besonders vorteilhaft.
Der schweißhemmende Wirkstoff ist in den erfindungsgemäßen Zusammensetzungen in einer Menge von 2 - 30 Gew.-%, vorzugsweise 5 - 25 Gew.-% und insbesondere 10 - 20 Gew.-%, bezogen auf die Menge der Aktivsubstanz in der gesamten flüssigen Zusammensetzung, enthalten.

Die erfindungsgemäßen Zusammensetzungen enthalten mindestens ein mit den transpirationshemmenden Verbindungen kompatibles organisches Polymer mit verdickender Wirkung. Mit den kationischen transpirationshemmenden Verbindungen kompatibel sind vor allem die nichtionischen, kationischen und amphoteren Polymere. Bevorzugt sind die nichtionischen Polymere, insbesondere die nichtionischen Polysaccharide. Erfindungsgemäß geeignete nichtionische Polysaccharide sind aus mehr als zehn Monosaccharideinheiten zusammengesetzt. Bevorzugte nichtionische Polysaccharide sind nichtionische Cellulose-Derivate, wie Methylcellulose, Hydroxypropylcellulose, Methylhydroxypropylcellulose oder Hydroxyethylcellulose. Ebenfalls geeignet sind die aus α-D-Glucose-Einheiten aufgebauten Stärken sowie Stärkeabbauprodukte wie Amylose, Amylopektin und Dextrine. Erfindungsgemäß vorteilhaft sind chemisch und/oder thermisch modifizierte Stärken, z. B. Hydroxypropylstärkephosphat, Dihydroxypropyldistärkephosphat oder die Handelsprodukte Dry Flo®.
Als kationische Polymere geeignet sind kationische Cellulose-Derivate, z. B. die Handelsprodukte Celquat® und Polymer JR®, und bevorzugt Celquat® H 100, Celquat® L 200 und Polymer JR® 400 (Polyquatemium-10) sowie Polyquaternium-24.
Die verdickenden Polymere sind in den erfindungsgemäßen Zusammensetzungen in einer Menge von 0,02 - 1,5 Gew.-%, vorzugsweise 0,05 - 1,0 Gew.-% und insbesondere 0,1 - 0,5 Gew.-%, bezogen auf die Menge der Aktivsubstanz in der gesamten flüssigen Zusammensetzung, enthalten.

Weiterhin enthalten die erfindungsgemäßen Zusammensetzungen mindestens ein wasserlösliches Polyol, ausgewählt aus wasserlöslichen Diolen, Triolen und höherwertigen Alkoholen sowie Polyethylenglycolen. Unter Wasserlöslichkeit versteht man erfindungsgemäß, dass sich wenigstens 5 Gew.-% des Polyols bei 20 °C klar lösen oder aber - im Falle langkettiger oder polymerer Polyole - durch Erwärmen der Lösung auf 50 °C bis 60 °C in Lösung gebracht werden können. Unter den Diolen eignen sich C₂-C₁₂-Diole, insbesondere 1,2-Propylenglycol, Dipropylenglycol, Tripropylenglycol, Butylenglycole wie z. B. 1,2-Butylenglycol, 1,3-Butylenglycol und 1,4-Butylenglycol, Pentandiole, z. B. 1,2-Pentandiol, sowie Hexandiole, z. B. 1,6-Hexandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG) mit einem durchschnittlichen Molekulargewicht von 100 bis 1.000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit.
Die erfindungsgemäßen Zusammensetzungen enthalten das wasserlösliche Polyol in Mengen von 1 - 30 Gew.-%, bevorzugt 2 - 15 Gew.-% und besonders bevorzugt 3 - 10 Gew.-%, jeweils bezogen auf die gesamte flüssige Zusammensetzung.

Weiterhin enthalten die erfindungsgemäßen Zusammensetzungen mindestens ein Salz, asugewählt aus Natriumchlorid und Kaliumchlorid. Die erfindungsgemäßen Zusammensetzungen enthalten Natriumchlorid und/oder Kaliumchlorid in Mengen von 0,5 - 10 Gew.-%, bevorzugt 0,5 - 8 Gew.-% und besonders bevorzugt 1 - 6 Gew.-% und ganz besonders bevorzugt 3 - 5 Gew.-%, jeweils bezogen auf die gesamte flüssige Zusammensetzung.

In einer bevorzugten Ausführungsform enthalten die erfindungsgemäßen kosmetischen Zusammensetzungen 0,5 - 50 Gew.-%, bevorzugt 1 - 10 Gew.-%, besonders bevorzugt 4 - 8 Gew.-%, bezogen auf die gesamte Zusammensetzung, Ethanol oder/und Isopropanol.

In einer weiteren bevorzugten Ausführungsform enthalten die erfindungsgemäßen kosmetischen Zusammensetzungen mindestens einen wasserlöslichen und/oder öllöslichen hautpflegenden Wirkstoff.

In einer weiteren bevorzugten Ausführungsform sind die erfindungsgemäßen kosmetischen Zusammensetzungen frei von cyclischen Silicon-Ölen.

In einer bevorzugten Ausführungsform sind die erfindungsgemäßen kosmetischen Zusammensetzungen in einer transparenten oder transluzenten Verpackung verpackt. Geeignete Verpackungen sind Glasflaschen oder Kunststoffflaschen mit einer für Rollon-Applikationen geeigneten Kugel. Weiterhin kann die Verpackung eine geschriebene Anweisung enthalten, gemäß der das Produkt vor dem Gebrauch zu schütteln ist.

Die erfindungsgemäßen Zusammensetzungen können optional Deodorantien enthalten. Als Deodorantien geeignet sind Duftstoffe, antimikrobielle, antibakterielle oder keimhemmende Stoffe, enzymhemmende Stoffe, Antioxidantien und Geruchsadsorbentien. Geeignete antimikrobielle, antibakterielle oder keimhemmende Stoffe sind insbesondere C₁-C₄-Alkanole, C₂-C₄-Alkandiole, Organohalogenverbindungen sowie -halogenide, quartäre Ammoniumverbindungen, eine Reihe von Pflanzenextrakten und Zinkverbindungen. Bevorzugt sind halogenierte Phenolderivate wie z. B. Hexachlorophen oder Irgasan DP 300 (Triclosan, 2,4,4'-Trichlor-2' hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Zu diesen zählen viele ätherische Öle wie z. B. Nelkenöl (Eugenol), Minzöl (Menthol) oder Thymianöl (Thymol) sowie Terpenalkohole wie z. B. Farnesol. Auch aromatische Alkohole wie z. B. Benzylalkohol, 2-Phenylethanol oder 2-Phenoxyethanol können als Deodorant-Wirkstoffe eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Ebenfalls geeignet sind langkettige Diole, z. B. 1,2-Alkan-(C₈-C₁₈)-Diole, Glycerinmono-(C₆-C₁₆)-alkylether oder Glycerinmono(C₈-C₁₈)-Fettsäureester, die sehr gut haut- und schleimhautverträglich und gegen Corynebakterien wirksam sind.
Als enzymhemmende Stoffe sind vor allem solche desodorierend wirksam, die esterspaltende Enzyme inhibieren und auf diese Weise der Schweißzersetzung entgegenwirken. Hierfür eignen sich vor allem Zinksalze, Pflanzenextrakte, z.B. Citruskernextrakte, sowie die Ester von aliphatischen C₂-C₆-Carbonsäuren oder Hydroxycarbonsäuren und C₂-C₆-Alkoholen oder Polyolen, z. B. Triethylcitrat, Propylenglycollactat oder Glycerintriacetat (Triacetin).

Antioxidative Stoffe können der oxidativen Zersetzung der Schweißkomponenten entgegenwirken und auf diese Weise die Geruchsentwicklung hemmen. Geeignete Antioxidantien sind Aminosäuren (z. B. Glycin, Histidin, Tyrosin, Tryptophan) und deren Derivate, Imidazol und Imidazolderivate (z. B. Urocaninsäure), Peptide wie z. B. D,L-Carnosin, D-Carnosin, L-Camosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-; Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), ferner Metallchelatoren (z. B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und - sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate (z. B. Ferulasäure, Ethylferulat, Kaffeesäure), Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und Zink-Derivate (z. B. ZnO, ZnSO₄), Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden.

Als lipophile, öllösliche Antioxidantien aus dieser Gruppe sind Tocopherol und dessen Derivate, Gallussäureester, Flavonoide und Carotinoide sowie Butylhydroxytoluol/anisol bevorzugt. Als wasserlösliche Antioxidantien sind Aminosäuren, z. B. Tyrosin und Cystein und deren Derivate sowie Gerbstoffe, insbesondere solche pflanzlichen Ursprungs bevorzugt.
Die Gesamtmenge der Antioxidantien in den erfindungsgemäßen flüssigen Zusammensetzungen beträgt 0,001 - 10 Gew.-%, vorzugsweise 0,05 - 5 Gew.-% und insbesondere 0,05 - 2 Gew.-%, bezogen auf die gesamte flüssige Zusammensetzung. Als Geruchsabsorber können folgende Substanzen eingesetzt werden: Zinkricinoleat, Cyclodextrin und dessen Derivate, Hydroxypropyl-β-Cyclodextrin, weiterhin Oxide wie Magnesiumoxid oder Zinkoxid, wobei die Oxide nicht mit Aluminiumchlorhydrat kompatibel sind, weiterhin Stärke und Stärkederivate, Kieselsäuren, die ggf. modifiziert sein können, Zeolithe, Talcum sowie synthetische Polymere, z.B. Nylon.
Auch komplexbildende Stoffe können die desodorierende Wirkung unterstützen, indem sie die oxidativ katalytisch wirkenden Schwermetallionen (z. B. Eisen oder Kupfer) stabil komplexieren. Geeignete Komplexbildner sind z. B. die Salze der Ethylendiamintetraessigsäure oder der Nitrilotriessigsäure sowie die Salze der 1-Hydroxyethan-1,1-diphosphonsäure.

Die nachfolgenden Beispiele sollen die Erfindung verdeutlichen, ohne sie hierauf zu beschränken.

Es werden verschiedene Zweiphasen-Produkte hergestellt, die Ölphase wird z.B. zartgrün eingefärbt.

### a) Ölphase oben zartgrün - Wasserphase unten klar

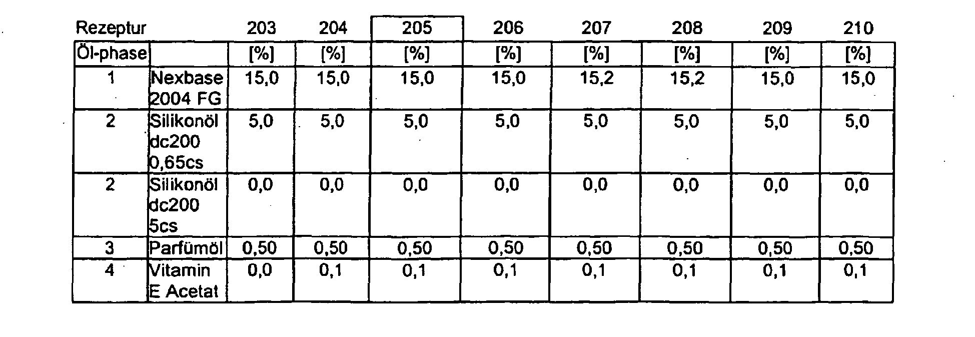

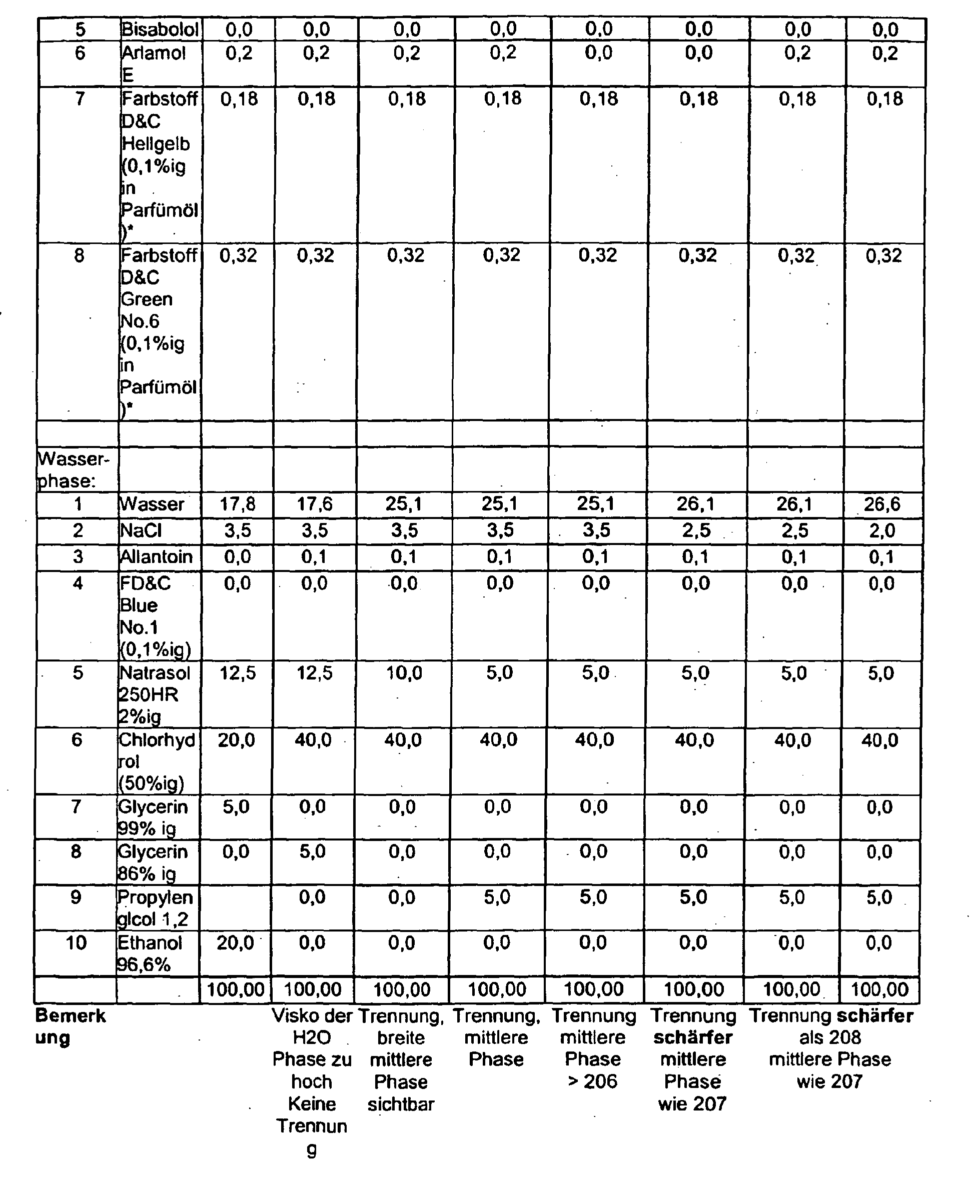

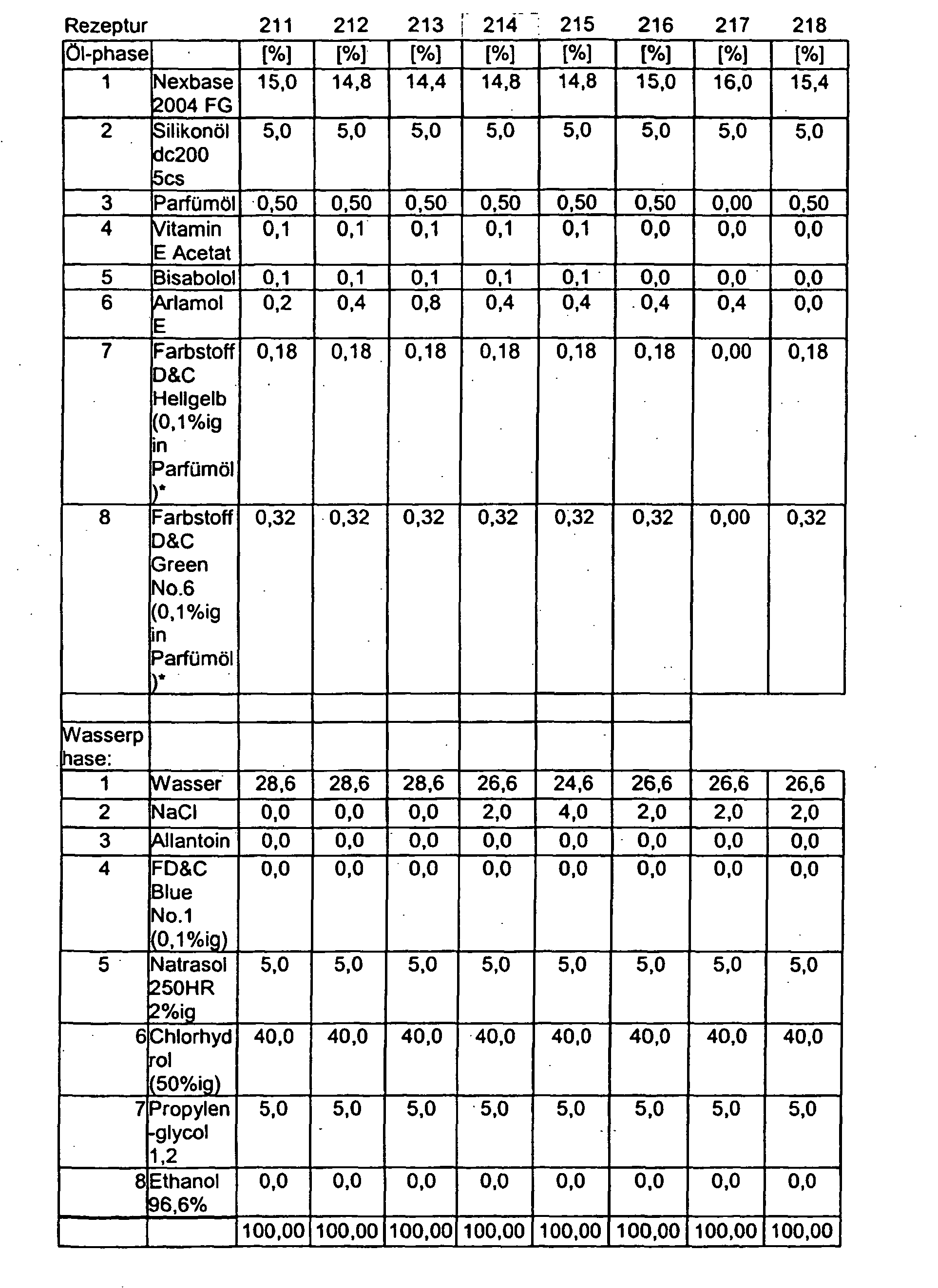

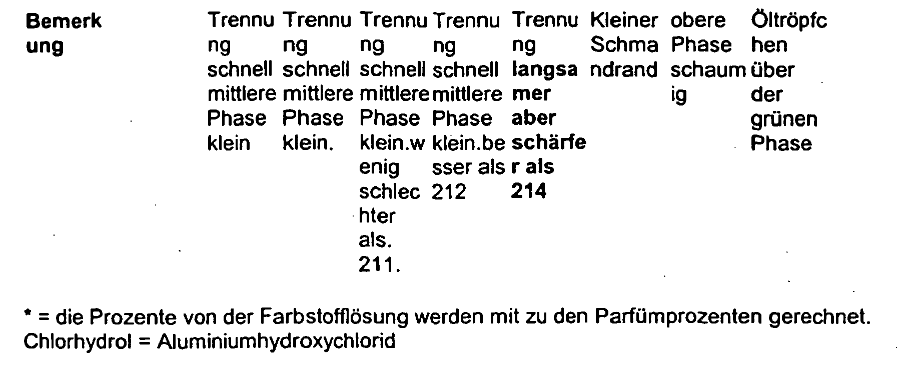

Die Mengenangaben in Gew.-% beziehen sich auf den Gehalt an Aktivsubstanz, bezogen auf die Gesamtzusammensetzung.

### Herstellungsverfahren:

### Wasserphase:

Zuerst wird die 2%ige Natrasol-Quellung hergestellt. Dazu wird das Wasser in einem Becherglas vorgelegt, das Natrasol Pulver vorsichtig eingestreut (Vermeidung von Klumpenbildung) und zur besseren Quellung 1-2 Tropfen NaOH (50%ig) zugegeben. Der Ansatz wird ca. 1 Stunde homogenisiert. Es werden nacheinander folgende Rohstoffe in einem Becherglas eingewogen und mit einem Flügelrührer verrührt: NaCl wird in Wasser gelöst, danach werden ggf. hautpflegende Zusätze und der Farbstoff zugeben. Die 2%ige Natrasol-Quellung wird hinzugefügt und etwas länger gerührt, bis eine homogene Mischung entstanden ist. Dann werden nacheinander Locron L, Glycerin/Propylenglycol und ggf. Ethanol zugefügt. Anschließend wird gerührt, bis die Mischung vollständig homogen ist.

### Ölphase:

In einem zweiten Becherglas wird die Ölphase eingewogen. Es werden nacheinander Nexbase 2004 FG und das Silikonöl in einem Becherglas eingewogen und mit einem Magnetrührer verrührt. Anschließend wird eine Vormischung aus Parfümöl, Arlamol E und ggf. dem Farbstoff zugefügt und alles homogenisiert. Danach werden beide Phasen in geeignete Applikations-Flaschen eingefüllt.

## Patentansprüche

1. Kosmetische Antitranspirant-Zusammensetzung, enthaltend eine Ölphase und eine Wasserphase, die im Ruhezustand nicht-emulgiert und durch eine Phasengrenze voneinander getrennt vorliegen, durch kurzzeitiges Schütteln emulgierbar sind, wobei die vollständige Entmischung im Ruhezustand innerhalb von 5 Sekunden bis 24 Stunden erfolgt, **gekennzeichnet durch** einen Gehalt an
c) 15 - 50 Gew.-% Ölphase, deren einzelne Komponenten miteinander eine klare Mischung bilden, enthaltend
- mindestens ein kosmetisches, bei 25° C flüssiges Öl,
- mindestens ein Parfümöl,
- 0,01 - 2 Gew.-% eines nichtionischen, kationischen, zwitterionischen oder amphoteren Emulgators,
d) 50 - 85 Gew.-% wässrige Phase, enthaltend
- 2 - 30 Gew.-%, mindestens einer Aluminium-, Zirkon-, Zink- oder TitanVerbindung mit transpirationshemmender Wirkung,
- mindestens ein mit den transpirationshemmenden Verbindungen kompatibles organisches Polymer mit verdickender Wirkung,
- mindestens ein wasserlösliches Polyol,
- 0,5 - 10 Gew.-% Natrium- oder Kaliumchlorid,
wobei sich alle Mengenangaben in Gew.-% auf das Gewicht der Gesamtzusammensetzung beziehen.

2. Kosmetische Zusammensetzung gemäß Patentanspruch 1, **dadurch gekennzeichnet, dass** der nichtionische Emulgator ausgewählt ist aus Ethylenoxid-Addukten von linearen C₃-C₂₂Alkoholen mit einer mittleren Anzahl der Ethylenoxid-Einheiten von 1 - 30 Ethylenoxid-Addukten von verzweigten C₃-C₂₈-Alkoholen mit einer mittleren Anzahl von Ethylenoxid-Einheiten von 1 - 30, Ethylenoxid-Propylenoxid-Mischaddukten von linearen C₃-C₂₂-Alkoholen mit einer mittleren Anzahl an Ethylenoxid-Einheiten von 2 - 50 und einer mittleren Anzahl an Propylenoxid-Einheiten von 2 - 35 und Propylenoxid-Addukten von linearen C₃-C₂₂-Alkoholen mit einer mittleren Anzahl der Propylenoxid-Einheiten von 1 - 30.

3. Kosmetische Zusammensetzung gemäß einem der vorstehenden Ansprüche, **dadurch gekennzeichnet, dass** 0,5 - 50 Gew.-%, bezogen auf die gesamte Zusammensetzung, Ethanol oder/und Isopropanol enthalten ist.

4. Kosmetische Zusammensetzung gemäß einem der vorstehenden Ansprüche, **dadurch gekennzeichnet, dass** mindestens ein wasserlöslicher und/oder öllöslicher hautpflegender Wirkstoff enthalten ist.

5. Kosmetische Zusammensetzung gemäß einem der vorstehenden Ansprüche, **dadurch gekennzeichnet, dass** mindestens ein lineares Silicon-Öl enthalten ist.

6. Kosmetische Zusammensetzung gemäß einem der vorstehenden Ansprüche, **dadurch gekennzeichnet, dass** die Zusammensetzung frei ist von cyclischen Silicon-Ölen.

7. Kosmetische Zusammensetzung gemäß einem der vorstehenden Ansprüche, **dadurch gekennzeichnet, dass** das organische Polymer mit verdickender Wirkung aus nichtionischen Polymeren, insbesondere nichtionischen Cellulosederivaten, ausgewählt ist.

8. Kosmetische Zusammensetzung gemäß einem der vorstehenden Ansprüche, **dadurch gekennzeichnet, dass** die Zusammensetzung in einer transparenten oder transluzenten Verpackung verpackt ist.

9. Kosmetische Zusammensetzung gemäß Anspruch 8, **dadurch gekennzeichnet, dass** die Verpackung eine geschriebene Anweisung enthält, gemäß der das Produkt vor dem Gebrauch zu schütteln ist.

## Claims

1. Cosmetic antiperspirant composition comprising an oil phase and a water phase which, in the resting state, are not emulsified and are separated from one another by a phase boundary, are emulsifiable by short-term shaking, where complete separation takes place in the resting state over the course of 5 seconds to 24 hours, **characterized by** a content of
c) 15-50% by weight of oil phase whose individual components form a clear mixture with one another comprising
- at least one cosmetic oil which is liquid at 25°C,
- at least one perfume oil,
- 0.01-2% by weight of a nonionic, cationic, zwitterionic or amphoteric emulsifier,
d) 50-85% by weight of aqueous phase, comprising
- 2-30% by weight of at least one aluminium, zirconium, zinc or titanium compound with an antiperspirant effect,
- at least one organic polymer with a thickening effect which is compatible with the antiperspirant compounds,
- at least one water-soluble polyol,
- 0.5-10% by weight of sodium chloride or potassium chloride,
where all of the quantitative data in % by weight refer to the weight of the total composition.

2. Cosmetic composition according to Patent Claim 1, **characterized in that** the nonionic emulsifier is chosen from ethylene oxide adducts of linear C₃-C₂₂-alcohols with an average number of ethylene oxide units of 1-30, ethylene oxide adducts of branched C₃-C₂₈-alcohols with an average number of ethylene oxide units of 1-30, ethylene oxide- propylene oxide mixed adducts of linear C₃-C₂₂₋alcohols with an average number of ethylene oxide units of 2-50 and an average number of propylene oxide units of 2-35 and of propylene oxide adducts of linear C₃-C₂₂-alcohols with an average number of propylene oxide units of 1-30.

3. Cosmetic composition according to one of the preceding claims, **characterized in that** 0.5-50% by weight, based on the total composition, of ethanol and/or isopropanol is present.

4. Cosmetic composition according to one of the preceding claims, **characterized in that** at least one water-soluble and/or oil-soluble skin care active ingredient is present.

5. Cosmetic composition according to one of the preceding claims, **characterized in that** at least one linear silicone oil is present.

6. Cosmetic composition according to one of the preceding claims, **characterized in that** the composition is free from cyclic silicone oils.

7. Cosmetic composition according to one of the preceding claims, **characterized in that** the organic polymer with a thickening effect is chosen from nonionic polymers, in particular nonionic cellulose derivatives.

8. Cosmetic composition according to one of the preceding claims, **characterized in that** the composition is packaged in a transparent or translucent packaging.

9. Cosmetic composition according to Claim 8, **characterized in that** the packaging contains a written instruction according to which the product is to be shaken prior to use.

## Revendications

1. Composition cosmétique antitranspirante contenant une phase huileuse et une phase aqueuse qui, à l'état de repos, ne sont pas émulsifiées et sont séparées l'une de l'autre par une interface, et que l'on peut émulsifier via une agitation de courte durée, la démixtion complète ayant lieu à l'état de repos dans un laps de temps de 5 secondes à 24 heures, **caractérisée par** une teneur
a) en phase huileuse, à concurrence de 15 à 50 % en poids, dont les composants individuels forment ensemble un mélange clair contenant
- au moins une huile cosmétique liquide à 25 °C ;
- au moins une huile de parfum ;
- à concurrence de 0,01 à 2 % en poids, un émulsifiant non ionique, cationique, zwitterionique ou amphotère ;
b) en phase aqueuse à concurrence de 50 à 85 % en poids, contenant
- à concurrence de 2 à 30 % en poids, au moins un composé d'aluminium, de zirconium, de zinc ou de titane possédant un effet inhibiteur de la transpiration ;
- au moins un polymère organique compatible avec les composés inhibiteurs de la transpiration, ayant un effet épaississant ;
- au moins un polyol soluble dans l'eau ;
- à concurrence de 0,5 à 10 % en poids, du chlorure de sodium
ou de potassium ;
toutes les proportions en % en poids se rapportant au poids de la composition totale.

2. Composition cosmétique selon la revendication 1, **caractérisée en ce que** l'émulsifiant non ionique est choisi parmi le groupe comprenant des adduits d'oxyde d'éthylène d'alcools linéaires en C₃-C₂₂ dont le nombre moyen des unités d'oxyde d'éthylène s'élève de 1 à 30, des adduits d'oxyde d'éthylène d'alcools ramifiés en C₃-C₂₈ dont le nombre moyen des unités d'oxyde d'éthylène s'élève de 1 à 30, des adduits mixtes d'oxyde d'éthylène-oxyde de propylène d'alcools linéaires en C₃-C₂₂ dont le nombre moyen des unités d'oxyde d'éthylène s'élève de 2 à 50 et dont le nombre moyen des unités d'oxyde de propylène s'élève de 2 à 35, et des adduits d'oxyde de propylène d'alcools linéaires en C₃-C₂₂ dont le nombre moyen des unités d'oxyde de propylène s'élève de 1 à 30.

3. Composition cosmétique selon l'une quelconque des revendications précédentes, **caractérisée en ce qu'**elle contient, à concurrence de 0,5 à 50 % en poids, rapportés à la composition totale, de l'éthanol et/ou de l'isopropanol.

4. Composition cosmétique selon l'une quelconque des revendications précédentes, **caractérisée en ce qu'**elle contient au moins une substance active pour les soins de la peau, soluble dans l'eau et/ou soluble dans l'huile.

5. Composition cosmétique selon l'une quelconque des revendications précédentes, **caractérisée en ce qu'**elle contient une huile de silicone linéaire.

6. Composition cosmétique selon l'une quelconque des revendications précédentes, **caractérisée en ce que** la composition est exempte d'huiles de silicone cycliques.

7. Composition cosmétique selon l'une quelconque des revendications précédentes, **caractérisée en ce que** le polymère organique ayant un effet épaississant est choisi parmi le groupe des polymères non ioniques, en particulier des dérivés non ioniques de la cellulose.

8. Composition cosmétique selon l'une quelconque des revendications précédentes, **caractérisée en ce que** la composition est conditionnée dans un emballage transparent ou translucide.

9. Composition cosmétique selon la revendication 8, **caractérisée en ce que** l'emballage contient une inscription indiquant que le produit doit être agité avant l'emploi.
